Europäisches Patentamt

European Patent Office

Office européen des brevets

(11) Publication number: **0 213 809 B1**

# EUROPEAN PATENT SPECIFICATION

(45) Date of publication of patent specification: **18.03.92** (51) Int. Cl.[5]: **A61K 35/78**

(21) Application number: **86306109.9**

(22) Date of filing: **07.08.86**

The file contains technical information submitted after the application was filed and not included in this specification

(54) **Extract of swertia japonica makino and method of obtaining the same.**

(30) Priority: **12.08.85 JP 175739/85**

(43) Date of publication of application:
**11.03.87 Bulletin 87/11**

(45) Publication of the grant of the patent:
**18.03.92 Bulletin 92/12**

(84) Designated Contracting States:
**DE FR GB**

(56) References cited:
**EP-A- 0 093 520**
**US-A- 3 133 864**

**PATENT ABSTRACTS OF JAPAN, vol. 8, no.
172 (C-237)[1609], 9th August 1984; & JP-A-59
70 605 (HIROSHI DOI) 21-04-1984**

**CHEMICAL ABSTRACTS, vol. 73, no. 8, 24th
August 1970, pages 223,224, abstract no.
38533w, Columbus, Ohio, US; & JP-A-70 10
517 (TOKYO PHARMACEUTICAL DEVELOP-
MENT CO., LTD) 15-04-1970**

(73) Proprietor: **KABUSHIKI KAISHA VITAMIN KEN-
KYUSHO
2134-1 Mitake Aza-Hirashiba
Mitake-cho Kani-gun Gifu-ken(JP)**

(72) Inventor: **Kurono, Masayasu
3-6-7, Sasao-nishi Toin-cho
Inabe-gun Mie-ken(JP)**
Inventor: **Iida, Takafumi
2-1-76, Ishiodai
Kasugai-shi Aichi-ken(JP)**
Inventor: **Kato, Eiichi
3-36, Sennaritori Nakamura-ku
Nagoya-shi Aichi-ken(JP)**
Inventor: **Yamakawa, Hidehumi
2-5-1, Gejo-cho
Kasugai-shi Aichi-ken(JP)**
Inventor: **Yagi, Kunio
2-21, Nishizato-cho Meito-Ku
Nagoya-shi Aichi-ken(JP)**

(74) Representative: **Dixon, Donald Cossar et al
Gee & Co. Chancery House Chancery Lane
London WC2A 1OU(GB)**

Rank Xerox (UK) Business Services

BIOLOGICAL ABSTRACTS, vol. 78, 1984, ref. no. 45, Biological Abstracts, Inc., Philadelphia, US; M. KUBOTA et al.: "Evaluation of cultivated Swertia Japonica: 1. Variation in the contents of various components during the efflorescence", & SHOYAKUGAKU ZASSHI, 37(3), 229-236, 1983

BIOLOGICAL ABSTRACTS, vol. 67, 1979, ref. no. 75300, Biological Abstracts, Inc., Philadelphia, US; J. YAMAHARA et al.: "Biologically active principles of crude drugs: pharmacological actions of Swertia Japonica extracts, swertiamarin and gentianine", & YAKUGAKU ZASSHI, 98(11), 1446-1451, 1978

CHEMICAL ABSTRACTS, vol. 104, no. 6, 10th February 1986, page 382, abstract no. 39718f, Columbus, Ohio, US; & JP-A-60 126 218 (OHYO SEIKAGAKU KENKYUSHO K.K.) 05-07-1985

**Description**

The present invention relates to a method of obtaining an extract of Swertia japonica. Makino and an use as defined in the claims.

Swertia japonica Makino is a biennial herb of the family Gentianaceae and has been well known as a folk medicine or galenical to use e.g. as a stomachic remedy or anodyne. Extracts of the herb are known to have a pharmacological action on stomach ulcers, and as analgesic, antiphlogistic, depressor, antibiotic and antileukemia, but components showing such actions have not been elucidated except for bitter one such as swertiamarin, which is a seco-iridoid glycoside, and thus various studies have been made to find the relation between the pharmacological actions and active components.

Recently, it has been reported in Jap. Unexamined Pat. Appln. Gazette No. 201713/1983 that oleanane-type triterpene compounds including oleanolic acid which is a component of Swertia japonica Makino have anti-ulcer and analgesic actions. The present inventors have found that oleanolic acid extracted and isolated from Swertia japonica Makino has an inhibiting action on the enzyme activity of 5 $\alpha$-reductase which has been considered to have a certain relation to alopecia, and have reported the same in Jap. Pat. Appln. No. 232723/1983 (Jap. Unexamined Pat. Appln. Gazette No. 126218/1985). Further, it has also been reported in the Japanese Journal "Igaku-Kenkyu (Medical Study") Vol. 30, page 766 (1960) that an extract of Swertia japonica Makino, which contains bitter glycosides such as swertiamarin, produces angiotelectasis.

An article in Nagano Res. Inst Health Pollution 1978 (1983), by Kubota et. al, abstracted in Biol. Abstr . 78 (1984) No 45, described extraction of S. japonica to yield swertiamarin, oleanolic acid and other components which were measured by chromatography; no details of the extractions method, amounts extracted or use of the extracts are given.

JP-A-59-70605 (see Patent Abstracts of Japan, 8, No 172 (1984)) discloses extracting an unidentified species of Swertia with hot oil or hot water for use as a hair tonic to assist growing hair on bald areas; no details of the extraction method of components or the extract are given.

US-A-3133864 discloses a process of extracting dried S. japonica Makino with boiling ethanol to obtain an extract of Swertiamarin and other bitter components, for use as a hair tonic.

Swertia japonica Makino contains various physiological active substances which may be classified into water-soluble components including seco-iridoid glycosides and flavone glycosides as well as water-insoluble components including triterpene compounds and xanthone compounds.

Since extraction from Swertia japonica Makino has been generally carried out with water, hot water or an alcohol solution having a concentration of less than 50 volume %, both water-soluble and water-insoluble components have not been extracted concurrently and sufficiently and more particularly, water-insoluble components have not sufficiently been extracted. The extracts of Swertia japonica Makino prepared for medicines have different compositions from one another, depending on the conditions of extraction.

In order to overcome the disadvantages referred to, we have sought a convenient method for the preparation of an extract of Swertia japonica Makino with special attention to the following:-
(1) To extract useful components so that the original composition in Swertia japonica Makino is maintained in composition of the extract, and
(2) To adequately extract both water-soluble and water-insoluble components as well as minor components.

The inventors have studied extraction solvents and found that less polar solvents such as diethyl ether are not preferable, because those increase the extraction rate of water-insoluble compounds, but not of the water-soluble swertiamarin, and that when an alcohol is employed, the composition of the extract changes depending on the kind of alcohol and its concentration. For instance, when 70 volume % methanol aqueous solution is employed, the content of swertiamarin (water-soluble) can be made higher in the extract, but the content of oleanolic acid (water-insoluble) becomes lower, reducing the ratio of oleanolic acid to swertiamarin. When the concentration of ethanol is less than 60 volume %, it is difficult to extract oleanolic acid, and the extraction rate of the component becomes lower, as the concentration of ethanol decreases. From these results it has been concluded that 70 to 90 volume % ethanol aqueous solution is most preferable as the solvent for extraction.

When the solvent is used in an amount of not higher than 5 times volume per weight of dried Swertia japonica Makino, a large amount of extract can be obtained, but the resultant extract is turbid and the content of oleanolic acid in the extract, per weight of Swertia japonica Makino, is low, and we have found that 6 to 20 times volume of the solvent is required to increase the content of oleanolic acid in the extract.

Therefore, a method according to the invention for obtaining the extract of Swertia japonica Makino comprises extraction with a 70 to 90 volume % ethanol aqueous solution in an amount of 6 to 20 times volume per weight of dried Swertia japonica Makino.

Extraction with 10 times the volume of the solvent is preferable, taking into consideration the efficiency of extraction and the cost of solvent for the application of the method to an industrial scale and the avoidance of turbidity of the extract.

In practice, the extraction is carried out by soaking or immersing Swertia japonica Makino, for instance dried and finely cut, in an ethanol aqueous solution having said concentration and amount for about 24 hours at room temperature, and then subjecting the mixture to filtration or centrifugal separation to obtain the extract. High efficiency of extraction can be obtained by stirring during the soaking and it is convenient to repeat the extraction twice or more. The extraction can be carried out in a batch or continuous manner.

The resulting extract obtained according to the invention comprises both water-soluble swertiamarin and water-insoluble oleanolic acid, in a concentration of at least 20 mg swertiamarin and 6 mg oleanolic acid in the extract obtained from 1 g of dried Swertia japonica Makino.

The extract obtained according to the invention is suitable for use especially as an effective agent for the prevention of premature alopecia.

The invention will now be further explained with reference to Examples, Reference Examples and Test Example.

Example 1

5g of finely cut dried Swertia japonica Makino or coarse pieces thereof were soaked in 50ml of 70 volume % ethanol aqueous solution to stand for 24 hours at room temperature and then filtered to obtain a primary extract. To the residue, ethanol aqueous solution having the concentration and amount of the same as above was added to repeat the treatment as above to obtain a secondary extract. Two extracts were combined (Yield: 93.0ml).

Example 2

5g of finely cut dried Swertia japonica Makino or coarse pieces thereof were soaked in 50ml of 80 volume % ethanol aqueous solution to stand for 24 hours at room temperature and then filtered to obtain a primary extract. To the residue, ethanol aqueous solution having the concentration and amount of the same as above was added to repeat the treatment as above to obtain a secondary extract. Two extracts were combined (Yield: 94.0ml).

Example 3

5g of finely cut dried Swertia japonica Makino or coarse pieces thereof were soaked in 50ml of 90 volume % ethanol aqueous solution to stand for 24 hours at room temperature and then filtered to obtain a primary extract. To the residue, ethanol aqueous solution having the concentration and amount of the same as above was added to repeat the treatment as above to obtain a secondary extract. Two extracts were combined (Yield: 95.0ml).

Reference Example 1

5g of finely cut dried Swertia japonica Makino or coarse pieces thereof were soaked in 50ml of 50 volume % ethanol aqueous solution to stand for 24 hours at room temperature and then filtered to obtain a primary extract. To the residue, ethanol aqueous solution having the concentration and amount of the same as above was added to repeat the treatment as above to obtain a secondary extract. Two extracts were combined (Yield: 91.5ml)

Reference Example 2

5g of finely cut dried Swertia japonica Makino or coarse pieces thereof were soaked in 50ml of 60 volume % ethanol aqueous solution to stand for 24 hours at room temperature and then filtered to obtain a primary extract. To the residue, ethanol aqueous solution having the concentration and amount of the same as above was added to repeat the treatment as above to obtain a secondary extract. Two extracts were combined (Yield: 93.5ml).

Reference Example 3

5g of finely cut dried Swertia japonica Makino or coarse pieces thereof were soaked in 25 ml of 70 volume % ethanol aqueous solution to stand for 24 hours at room temperature and then filtered to obtain a primary extract. To the residue, ethanol aqueous solution having the concentration and amount of the same as above was added to repeat the treatment as above to obtain a secondary extract. Two extracts were combined (Yield: 45.0ml).

Reference Example 4

5g of finely cut dried Swertia japonica Makino or coarse pieces thereof were soaked in 50ml of 70 volume % methanol aqueous solution to stand for 24 hours at room temperature and then filtered to obtain a primary extract. To the residue, methanol aqueous solution having the concentration and amount of the same as above was added to repeat the treatment as above to obtain a secondary extract. Two extracts were combined (Yield: 91.5ml).

Reference Example 5

5g of finely cut dried Swertia japonica Makino or coarse pieces thereof were soaked in 50ml of diethyl ether to stand for 24 hours at room temperature and then filtered to obtain a primary extract. To the residue, diethyl ether in the amount of the same as above was added to repeat the treatment as above to obtain a secondary extract. Two extracts were combined (Yield: 71.0ml).

Test Example

Assay for the amounts of swertiamarin and oleanolic acid was performed by high-performance liquid chromatography and gas chromatography, respectively, under the following conditions.

Swertiamarin:
HPLC column ;    Silica gel ODS (4.6 x 250mm)
Solvent ;    $CH_3CN/H_2O$ (10 : 90)
Detector ;    UV (238nm)
Flow rate ;    1 ml/min
Detection limit;    10 $\mu$g/ml
Oleanolic acid
Pre-treatment ;    Trifluoroacetic acid anhydride, reacted with hexafluoro-2-propanol
Column ;    2% Silicone DCQF-1 on Uniport HP (3 x 2000mm)
Carrier gas ;    $N_2$
Flow rate ;    50 ml/min
Detector ;    FID
Dection limit;    2.5 $\mu$g/ml

Content of extract, extraction rate, contents of swertiamarin and of oleanolic acid, and ratio of oleanolic acid to swertiamarin in each extract obtained by the methods disclosed in Examples 1 to 3 as well as Reference Examples 1 to 5 are shown in Table 1.

The volume of extract, weight thereof, and amounts of swertiamarin and of oleanolic acid in each extract obtained from 1g of dried Swertia japonica Makino are shown in Table 2.

Contents of swertiamarin and oleanolic acid in commercially available hair tonics containing an extract of Swertia japonica Makino were measured and are shown in Table 3.

5

## TABLE 1

| | Content of extract (mg/ml) | Extraction rate (%) | Content of swertia- marin (mg/ml), (S) | Content of oleanolic acid (mg/ml), (0) | Ratio of 0/S |
|---|---|---|---|---|---|
| **Example** | | | | | |
| 1 | 17.3 | 32.2 | 3.74 | 0.654 | 0.175 |
| 2 | 16.0 | 30.0 | 3.43 | 0.660 | 0.192 |
| 3 | 13.9 | 26.4 | 2.98 | 0.652 | 0.219 |
| **Reference Example** | | | | | |
| 1 | 17.5 | 32.0 | 3.91 | 0.0509 | 0.0130 |
| 2 | 16.9 | 31.6 | 3.45 | 0.211 | 0.0612 |
| 3 (*) | 28.9 | 26.0 | 6.91 | 0.796 | 0.115 |
| 4 | 14.3 | 26.2 | 3.45 | 0.0660 | 0.0191 |
| 5 | 2.99 | 4.24 | 0.00487 | 0.841 | 173 |

* : In this Example,       turbid filtrate was obtained, and the value was estimated with the turbid solution.

EP 0 213 809 B1

TABLE 2

|  | Volume of extract (ml) | Amount of extract (mg) | Amount of swertiamarin (mg) | Amount of oleanolic acid (mg) |
|---|---|---|---|---|
| Example |  |  |  |  |
| 1 | 18.6 | 322 | 69.6 | 12.2 |
| 2 | 18.8 | 300 | 64.4 | 12.4 |
| 3 | 19.0 | 264 | 56.6 | 12.4 |
| Reference Example |  |  |  |  |
| 1 | 18.3 | 320 | 71.6 | 0.932 |
| 2 | 18.7 | 316 | 64.6 | 3.94 |
| 3 (*) | 9.00 | 260 | 62.2 | 7.16 |
| 4 | 18.3 | 262 | 63.2 | 1.21 |
| 5 | 14.2 | 42.4 | 0.0692 | 11.9 |

Note: Value given in this Table are obtained from 1g of dried <u>Swertia japonica</u> Makino

* : In this Example,      turbid filtrate was obtained, and the value was estimated with the turbid solution.

## TABLE 3

| | Content of swertiamarin ($\mu$g/ml) | Content of oleanolic acid ($\mu$g/ml) |
|---|---|---|
| S Company, N products | 24.0 | — |
| S Company, F products | — | — |
| N Company, H products | 365 | — |
| K Company, S products | 321 | — |

Note: Symbol "—" means not detectable

## Claims

1. A method of obtaining an extract of Swertia japonica Makino, which comprises extracting with a 70 to 90 volume % ethanol aqueous solution of 6 to 20 times volume per weight of dried japonica Makino.

2. A method as claimed in Claim 1, wherein the concentration of said ethanol aqueous solution is 70 volume %.

3. A method as claimed in Claim 1 or 2, wherein said ethanol aqueous solution is used in the amount of 10 times per weight of the dried japonica Makino.

4. Use of an extract of Swertia japonica Makino obtained by a method according to claim 1, 2 or 3, comprising swertiamarin and oleanolic acid extracted from Swertia japonica Makino, for the manufacture of compositions for the treatment of alopecia.

**Revendications**

1. Méthode pour obtenir un extrait de swertia japonica makino qui consiste à extraire avec une solution aqueuse d'éthanol à 70 à 90 % en volume de six à vingt fois le volume par poids de swertia japonica makino séchée.

2. Méthode selon la revendication 1, dans laquelle la concentration de la solution aqueuse d'éthanol est 70 % en volume.

3. Méthode selon la revendication 1 ou la revendication 2, dans laquelle la solution aqueuse d'éthanol est utilisée en la quantité de dix fois par poids de swertia japonica makino séchée.

4. Utilisation d'un extrait de swertia japonica makino obtenu par une méthode selon l'une des revendications 1 à 3, contenant de la swertiamarine et de l'acide oléanolique extraits de swertia japonica makino pour la fabrication de compositions pour le traitement de l'alopécie.

**Patentansprüche**

1. Verfahren zur Herstellung eines Extrakts aus Swertia Japonica Makino, umfassend die Extraktion mit einer 70 bis 90 Volumen %igen Äthylalkohol-Wasser-Lösung mit der 6-bis 20-fachen Menge des Gewichts der getrockneten Japonica Makino.

2. Verfahren nach Anspruch 1, dadurch gekennzeichnet, daß die Konzentration der Äthylalkohol-Wasser-Lösung 70 Volumen % beträgt.

3. Verfahren nach Anspruch 1 oder 2, dadurch gekennzeichnet, daß die Äthylalkohol-Wasser-Lösung in einer Menge benutzt wird, die dem 10-fachen Gewicht der getrockneten Japonica Makino entspricht.

4. Verwendung eines Extrakts von Swertia Japonica Makino gemäß dem Herstellungsverfahren nach einem der Ansprüche 1 bis 3, und umfassend Swertiamarin und Caryophyllin zur Herstellung von Kompositionen zur Behandlung von Haarausfall.